# FASCICULE DE BREVET EUROPEEN

(11) **EP 3 898 938 B1**
(45) Date de publication et mention de la délivrance du brevet: **05.07.2023**
(21) Numéro de dépôt: 19848890.0
(22) Date de dépôt: 19.12.2019
(51) Int. Cl.: C12N 1/20, A01N 63/00, C05F 11/08, C12R 1/38

(54) **SOUCHE BACTÉRIENNE BIOFERTILISANTE**
BAKTERIENSTAMM ALS BIODÜNGER
BIOFERTILIZING BACTERIAL STRAIN

(30) Priorité: 20.12.2018 FR 1873596
(43) Date de publication de la demande: 27.10.2021
(73) Titulaire: Université de Lorraine, 54000 Nancy (FR); Institut national de recherche pour l'agriculture l'alimentation et l'environnement, 75007 Paris (FR)
(72) Inventeur: SLEZACK-DESCHAUMES, Sophie, 55200 Commercy (FR); PIUTTI, Séverine, 54170 Barisey au Plain (FR); L'YVONNET, Pierre, 42120 Perreux (FR); ROSELLI, Sandro, 55200 Commercy (FR)
(74) Mandataire: Plasseraud IP
(86) Numéro de dépôt international: PCT/FR2019/053221
(87) Numéro de publication internationale: WO 2020/128374

(56) Documents cités:
- WO-A1-2014/163473
- CREGUT M ET AL: "Density, structure, and diversity of the cultivable arylsulfatase-producing bacterial community in the rhizosphere of field-grown rape and barley", SOIL BIOLOGY AND BIOCHEMISTRY, PERGAMON, OXFORD, GB, vol. 41, no. 4, 1 avril 2009 (2009-04-01), pages 704-710, XP026007555, ISSN: 0038-0717, DOI: 10.1016/J.SOILBIO.2009.01.005 [extrait le 2009-01-30]
- SUBRAMANIAM GOPALAKRISHNAN ET AL: "Plant growth-promoting traits of Pseudomonas geniculata isolated from chickpea nodules", 3 BIOTECH, vol. 5, no. 5, 1 octobre 2015 (2015-10-01) , pages 653-661, XP055610712, DE ISSN: 2190-572X, DOI: 10.1007/s13205-014-0263-4

## Description

### Domaine technique

La présente invention porte sur le domaine de la fertilisation des cultures, et particulièrement sur une souche bactérienne biofertilisante.

### Technique antérieure

La seconde moitié du XX^{ème} siècle s'est accompagnée d'une intensification importante de l'agriculture dans les pays développés. Cette intensification s'est largement appuyée sur le recours à un ensemble d'intrants qui ont permis de mieux maitriser les facteurs limitants la production agricole (Tilman et al., 2002). La généralisation de l'emploi des fertilisants chimiques explique pour beaucoup la croissance très rapide de la production agricole à partir des années 1950. Cependant, l'usage intensif de fertilisants minéraux azotés (N) a conduit au transfert de ces éléments vers les eaux de surface et souterraines et à l'émission de gaz à effet de serre qui contribuent à la destruction de la couche d'ozone (Binbradan et al., 2015 ; Sebilo et al., 2013). De plus, les fertilisants N ont contribué à altérer la diversité biologique dans les agrosystèmes, avec des conséquences sur le fonctionnement des écosystèmes (Tilman et al., 1997) et leur productivité primaire (Hector et al., 1999). Ces effets négatifs, inacceptables du point de vue sociétal, résultent majoritairement d'une médiocre efficience d'utilisation des engrais N par les plantes cultivées. Ainsi, de 20 à 80% de ces engrais seraient dispersés dans l'environnement plutôt que d'être absorbés et assimilés par les cultures (Bindraban et al., 2015). A ce jour, il devient donc nécessaire de limiter l'utilisation de fertilisants chimiques azotés.

Pour un élément comme le soufre (S), la problématique est différente. En effet, la mise en oeuvre depuis les années 1980 de directives internationales pour réduire les rejets industriels atmosphériques et la diminution de la teneur en S libre des fertilisants N, P, K a conduit paradoxalement à l'apparition de déficiences en S sur les cultures. Ces déficiences peuvent pénaliser les rendements et la qualité des récoltes. Il est donc nécessaire d'améliorer la fourniture de S pour les cultures, d'autant plus que S permet d'améliorer l'assimilation de N chez les plantes.

Il existe donc un réel besoin pour des solutions non-chimiques, s'appuyant sur des processus biologiques, et permettant d'améliorer la disponibilité des éléments nutritifs (éléments minéraux) du sol pour la plante (solution biofertilisante) et d'augmenter l'efficience de leur utilisation par la plante (solution phytostimulante). Ceci est particulièrement important concernant N et S dans la mesure où plus de 90% de N et S dans les sols sont sous des formes organiques qui doivent être transformées par les microorganismes du sol (processus de décomposition et minéralisation des matières organiques) pour libérer des formes minérales accessibles pour les plantes.

De nombreuses souches de Pseudomonas ayant des propriétés biofertilisantes sonts décrites dans l'art antérieur. Ces propriétés sont l'augmentation de l'activité arylsulfatase (Cregut et al., Soil Biology and Biochemistry, 2009, 41(4):704-710), la solubilisation de phosphore (WO 2014/163473) ou l'augmentation de l'azote (Gopalakrishnan et al., 3 Biotech, 2015, 5(5):653-661).

### Résumé de l'invention

La présente invention porte sur une souche bactérienne biofertilisante permettant de répondre aux besoins ci-dessus.

Ainsi, dans un premier aspect, la présente invention porte sur une souche bactérienne telle que déposée le 24 octobre 2018 à la Collection Nationale de Culture de Micro-organismes (CNCM), 28 rue du Dr. Roux, 75724 PARIS CEDEX 15, en vertu du traité de Budapest sous le numéro CNCM I-5372.

Selon un autre aspect, la présente invention porte sur une composition comprenant la souche bactérienne telle que déposée le 24 octobre 2018 à la Collection Nationale de Culture de Micro-organismes (CNCM), 28 rue du Dr. Roux, 75724 PARIS CEDEX 15, en vertu du traité de Budapest sous le numéro CNCM I-5372.

L'invention porte également sur un procédé de fertilisation, ledit procédé comprenant une étape dans laquelle la composition selon l'invention est appliquée à une plante ou à un milieu de culture comme un sol.

Dans un autre mode de réalisation, la présente invention porte également sur l'utilisation de la souche bactérienne mentionnée ci-dessus en tant que biofertilisant ou phytostimulant.

### Exposé de l'invention

Les présents inventeurs sont parvenus à identifier et isoler une souche bactérienne biostimulante qui présente des propriétés biofertilisantes remarquables et qui permet de résoudre les problèmes énoncés ci-dessus.

Les « **biostimulants** » sont définis comme étant des produits contenant une ou des substance(s) et/ou des microorganismes dont la fonction consiste, lors de l'application sur les plantes ou dans la rhizosphère, à stimuler les processus naturels au bénéfice de l'absorption des éléments nutritifs et/ou de l'efficacité accrue de leur utilisation, ainsi que la tolérance aux facteurs de stress abiotiques, indépendamment du contenu en éléments nutritifs des produits (Du Jardin, 2012). Selon les processus biologiques influencés par le biostimulant, celui-ci peut être qualifié de « phytostimulant », «biofertilisant», d' «activateur de la vie du sol» ou encore « phytoactivateur » (Faessel et Tostivint, 2016).

Les biostimulants comprennent donc des solutions à base de microorganismes (inoculants microbiens) ou de substances organiques de natures chimiques diverses (Calvo et al., 2014). Les inoculants microbiens comprennent des microorganismes libres (bactéries et champignons) et/ou des microorganismes symbiotiques (bactéries appartenant aux Rhizobiacées, champignons mycorhiziens). Certaines bactéries libres pouvant être utilisées en tant qu'inoculant microbien, peuvent être qualifiées de «PGPR » (pour Plant Growth Promoting Rhizobacteria) (Hayat et al., 2010).

Les inoculants microbiens, dont les PGPR, peuvent améliorer la croissance de la plante en augmentant la disponibilité d'éléments minéraux spécifiques dans le sol, à savoir le phosphore (P), le potassium (K), le fer (Fe), le calcium (Ca) et le magnésium (Mg) (Calvo et al., 2014). Les microorganismes agissent en i) solubilisant les éléments comme P via la production d'acides organiques, ii) minéralisant le P organique du sol via la production de phosphatases, iii) en produisant des sidérophores.

Une souche bactérienne « **biofertilisante** » a la capacité de stimuler le fonctionnement biologique du sol en lien avec la minéralisation des matières organiques et à améliorer la disponibilité en éléments minéraux, en particulier N, S voire P. Cette capacité peut résulter de la production d'enzymes microbiennes permettant la décomposition et la minéralisation d'éléments, comme par exemple une activité arylsulfatase qui permet la libération de soufre minéral ou encore des activités protéase/amino-peptidase qui permettent la libération d'azote.

Une souche bactérienne ayant la capacité de modifier les équilibres microbiens dans les sols et d'améliorer la minéralisation des matières organiques de manière globale est qualifiée « **d'activateur de la vie du sol** »**.**

Une souche bactérienne est par ailleurs dite « **phytostimulante** » lorsqu'elle a la capacité de stimuler la croissance, en particulier du système racinaire, des plantes dans leurs premiers stades de développement. Ce type de souche améliore donc les capacités d'exploration du sol et d'interception des éléments par la plante. Les souches phytostimulantes peuvent influencer le développement racinaire de la plante via la production d'analogues de phytohormones qui régulent l'initiation et l'élongation racinaire et la production de poils absorbants (Vacheron et al., 2013).

La souche bactérienne selon la présente invention présente une très forte activité biofertilisante. En effet, les présents inventeurs ont notamment démontré que l'inoculation de la souche bactérienne selon l'invention permettait d'augmenter significativement les activités enzymatiques du sol impliquées dans la décomposition et minéralisation de N et S (et particulièrement les activités protéases et arylsulfatases) et la teneur en minéraux (particulièrement N et S) du sol.

Les inventeurs ont également démontré que la souche selon la présente invention présente une activité phytostimulante. Ainsi, la souche permet d'influencer l'architecture du système racinaire des plantes et notamment d'augmenter la longueur et la surface des racines fines qui sont les racines responsables de la capture des minéraux dans le sol par la plante.

La souche bactérienne selon l'invention est la souche déposée le 24 octobre 2018 à la Collection Nationale de Culture de Micro-organismes (CNCM), 28 rue du Dr. Roux, 75724 PARIS CEDEX 15, en vertu du traité de Budapest sous le numéro CNCM-I-5372.

Après analyse (séquence partielle de l'ADN ribosomique 16S d'une longueur de 1450 pb amplifiée avec la Taq PrimeStar Max), il s'avère que la présente souche est affiliée au genre « *Pseudomonas* » et présente particulièrement 99% d'identité avec *Pseudomonas moraviensis* souche 1B4 (n° d'accession GenBank : NR 043314.1). Dans le cadre de la présente demande la souche bactérienne objet de l'invention peut donc également être désignée en tant que souche « P » ou « affiliée à *Pseudomonas* ».

La souche bactérienne selon la présente invention peut être formulée sous la forme d'une composition. Ainsi, dans un autre aspect, la présente invention porte sur une composition ou « inoculant », comprenant la souche bactérienne selon la présente invention.

La composition peut être sous forme de poudre, de granulés, de crème (ou « boue ») ou sous forme liquide (Bashan et al., inoculants of plant growth-promoting bacteria for use in agriculture ; Biotechnol Adv 16:729-770, 1998 ; Catroux et al., Trends in rhizobial inoculant production and use, Plant Soil 230:21-30, 2001). Dans un mode de réalisation préféré, la souche bactérienne selon l'invention est formulée sous forme liquide.

L'homme du métier connaît plusieurs moyens pour formuler des compositions bactériennes biofertilisantes, ou « inoculants » (voir par exemple Bashan, Yoav, et al. "Advances in plant growth-promoting bacterial inoculant technology: formulations and practical perspectives (1998-2013)." Plant and Soil 378.1-2 (2014): 1-33).

Afin de préparer une telle composition, la souche bactérienne sera typiquement intégrée à un matériau de support. Le support doit permettre la croissance de la bactérie selon l'invention, maintenir un niveau satisfaisant de bactéries viables pendant une période donnée, et permettre la libération de ces bactéries en quantités suffisantes pour permettre leur effet biostimulant (voir Date et al., « Advances in inoculant technology: a brief review», Aust J Exp Agr 41:321-325, 2001). Ce support peut être sous forme solide, liquide ou de gel. Les supports peuvent être divisés en cinq grandes catégories:
- les sols dont la tourbe, le charbon, le biochar, les argiles, et les sols inorganiques;
- les déchets organiques végétaux et animaux d'origine industrielle ou de l'agriculture
- les matériaux inertes tels que les polymères (tels que l'alginate ou le la chitosane) ou les fragments rocheux (comme la vermiculite et la perlite);
- les cultures microbiennes lyophilisées ou déshydratées incorporées dans un support solide ou utilisées en tant que telles ; et
- les supports liquides qui comprennent la souche bactérienne à formuler seule ou en présence de certains additifs permettant d'améliorer la stabilité, la dispersion ou la turbidité.

Le support peut être pré-stérilisé et/ou enrichi en éléments nutritifs tels que le saccharose, le maltose, le tréhalose, la mélasse, le glucose et le glycérol. Ceci permet d'augmenter la viabilité et la conservation de la composition comprenant la bactérie selon l'invention.

Les supports « sols » peuvent comprendre des additifs. Ces additifs sont typiquement choisis parmi la chitine, la pyrophyllite, le charbon, les roches sédimentaires comme le lignite, les sucres (monosaccharides, oligosaccharides et polysaccharides), la gomme arabique et les huiles minérales et organiques.

Les additifs utilisés pour des supports liquides sont typiquement choisis parmi le caboxyméthylcellulose, le glycérol, les sucres (monosaccharides tels le glucose, le galactose, le fructose ; oligosaccharides et polysaccharides tels le saccharose, le lactose, le maltose ou le tréhalose), le glycérol, l'EDTA ferrique, la gomme arabique et les huiles minérales et organiques.

La composition peut ne contenir que les bactéries selon l'invention, on parlera alors d'inoculant « primitif ».

Les compositions sous forme liquide comprennent en moyenne au moins 10⁶ CFU par mL. Ces inoculants sont généralement formulés en tant que suspension bactérienne dans une solution contenant de l'eau et/ou des huiles minérales et/ou des huiles organiques.

La composition peut également être sous forme solide telle que de la poudre ou des granulés. Selon cette formulation, les bactéries peuvent être encapsulées dans des matrices polymériques comme des billes/granules d'alginate. Typiquement, les bactéries sont formulées dans un support constitué de matériaux inertes (comme l'alginate) et le mélange est mis sous forme de granulés ou de poudre. La formulation peut être ensuite lyophilisée. Typiquement, la lyophilisation peut être effectuée en présence d'un cryoprotectant tel que le saccharose (par exemple présent dans une quantité entre 0 et 10%, spécifiquement 5% en poids).

La souche bactérienne ou la composition selon la présente invention peuvent être utilisées dans un procédé de fertilisation des sols. Ainsi, dans un autre aspect, la présente invention porte sur un procédé de fertilisation, ledit procédé comprenant une étape dans laquelle la composition ou la souche bactérienne selon l'invention est appliquée à une plante ou à milieu de culture tel que le sol.

Un « procédé de fertilisation » vise à apporter à un milieu de culture les éléments minéraux nécessaires au développement d'une plante. Le milieu de culture peut être le sol, mais également un support de culture (pour toutes les applications maraîchages que ce soit hors sol ou non) comme par exemple les terreaux, mais aussi les supports pour l'aquaponie, l'hydroponie ou l'aéroponie. Par « fertilisation » on entend donc ici aussi bien la fertilisation des cultures que l'amendement sur des sols non-cultivés.

Dans le cadre de la présente invention le sol peut être tout type de sol selon le système international de classification des sols (FAO-World Reference Base).

La « plante » peut être n'importe quel être vivant appartenant au règne végétal, qu'il soit cultivé ou pas. La plante est une plante terrestre. Elle peut être une plante ligneuse ou une plante herbacée. La plante peut, par exemple, être choisie parmi les Poacées, les légumineuses (*Fabaceae*) ou les plantes à graines oléagineuses.

Dans un mode de réalisation particulier, la plante est une céréale choisi dans le groupe constitué du maïs (*Zea mays L*), du riz (*Oryza sativa, Oryza glaberrina* ), des différentes espèces de blé (*Triticum aestivum* ; *Triticum spelta*; *Triticum durum* ; *Triticum dicoccum* ; *Triticum turgidum L.* subsp. *turanicum* ; *Triticum monococcum*), de l'orge (*Hordeum vulgare* L. subsp. *vulgare ; hordeum hexastichum*) ; du sorgho (*Sorghum bicolor*) ; de l'avoine (*Avena sativa*), du mil (*Pennisetum glaucum* ; *Panicum milliaceum* ; *Setaria italica* ; *Panicum sumatrense*), du seigle (*Secale cereale*), du triticale (*Triticosecale*).

Les « plantes » sont typiquement des plantes cultivées :
- les plantes cultivées en grandes cultures sont typiquement choisies parmi l'arachide, l'avoine de printemps et d'hiver, la betterave fourragère et industrielle, le blé tendre d'hiver et de printemps, le blé dur, le brome, le dactyle, l'épeautre, la fétuque, la fléole, le maïs grain, fourrager et ensilage, le millet, le miscanthus, le moha fourrage et grain, l'orge de printemps et d'hiver, le panic, le ray-grass anglais et italien, le riz, le seigle de printemps et d'hiver, le sorgho, le Switchgrass, le triticale de printemps et d'hiver, la cameline de printemps et d'hiver, le chanvre, le colza d'hiver et de printemps, le canola, la moutarde blanche et brune, de printemps et d'hiver, la navette, le pastel, la féverole de printemps et d'hiver, le lotier, le lupin, la luzerne, le pois de printemps et d'hiver, le pois fourrager et protéagineux, le sainfoin, le soja, le trèfle blanc, incarnat ou violet, la vesce, le houblon, le sarrasin, le lin printemps et d'hiver (oléagineux et textile), le tabac et le tournesol ;
- les plantes cultivées en jachères semées sont typiquement choisies parmi le ray-grass anglais et italien, le trèfle de Perse, la moutarde blanche, la phacélie, le trèfle blanc, incarnat ou violet et la vesce ;
- les plantes cultivées en cultures légumières sont typiquement choisies parmi l'ail, l'aneth, l'artichaut, l'asperge, l'aubergine, la betterave potagère, le brocolis, le cardon, la carotte, le céleri rave et branche, le cerfeuil tubéreux, la chicorée, le choux chinois, le choux de Bruxelles, le choux feuillus, le choux pommés, le choux fleurs, le choux raves, le cresson alénois, le cresson de terre, le crosne, le concombre, les cornichons, la courgette, le melon, la pastèque, le potimarron, le potiron, l'échalote, les épinards, le fenouil, la fève, le flageolet, le fraisier, les haricots, la laitue, les lentilles sèches et fraîches, la mâche, le maïs, le navet, le niébé, l'oignon, le panais, le persil, le piment, le poireau, les pois, le poivron, la pomme de terre, le pourpier, les radis, le raifort, la rhubarbe, la roquette, le rutabaga, les salsifis, la scarole frisée, la scorsonère, la tomate et le topinambour ;

- les plantes de cultures fruitières telles que le citronnier, le clémentinier, les limettes, le mandarinier, l'oranger, le pamplemoussier, le cognassier, le figuier, l'amandier, le châtaignier, le noisetier, le noyer, l'abricotier, le cerisier, le jujubier, le mirabellier, le nectarinier, le pêcher, le prunier, le kiwi, le nashi, le néflier, l'olivier, les airelles, le cassissier, le cynorhodon, le framboisier et autres Rubus, le groseillier, les mûres et mûriers, le myrtillier, le sureau noir, le poirier et le pommier;
- les plantes cultivées de culture porte-graine sont typiquement choisies parmi la betterave, le brome, le dactyle, la fétuque, le ray-grass, les légumineuses fourragères porte -graine, les ombellifères porte-graine, le fenouil porte-graine, la fève porte-graine, le lupin porte-graine, le lotier porte-graine, la luzerne porte-graine, le maïs porte-graine, le chanvre, le lin, le chrysanthème, la coloquinte, la giroflée, l'oeillet, la pensée, le pois de senteur, la reine marguerite, la rose trémière, la betterave, la carotte, le céleri, la chicorée annuelle et bisannuelle, le choux, la ciboulette, les cucurbitacées, l'échalote, les épinards, les haricots, la laitue, la mâche, le navet, l'oignon, le panais, le persil, le poireau, la poirée, le pois chiche, les pois, les radis, la roquette, l'arroche, l'aneth, le cerfeuil, la coriandre et le trèfle ;
- les plantes cultivées dans le cadre de cultures ornementales sont typiquement choisies parmi les conifères, les feuillus, les ormes, les liliacées, l'hippeastrum, le narcisse, la nérine, l'iris, la jacinthe, le lys, le muguet, le glaïeul, l'hortensia, l'oeillet, l'iris pâle, le palmier, le pélargonium, les rosiers les tulipes et les autres espèces florales ;
- les plantes cultivées dans les cultures tropicales sont typiquement l'ananas et autres anacardiacées, l'avocatier, le bananier, la canne à sucre, le fruit de la passion, le dachine, le manioc, la patate douce, le songe, l'igname, le manguier et le papayer ;
- les plantes cultivées en viticulture telles que le raisin de cuve ou de table (et notamment les différentes espèces de vignes correspondantes) ; et
- les plantes cultivées à des fins aromatiques, à parfum ou médicinales sont typiquement choisies parmi l'absinthe, l'achillée millefeuille, l'armoise, la balsamite, la bardane, l'aneth, la camomille romaine, les épices, l'angélique, le basilic, le carvi, le cerfeuil, la ciboulette, l'estragon, le laurier, le romarin, la menthe, le persil, la sauge, la gentiane, la lavande et le lavandin, la livèche officinale, l'onagre, l'origan, la bourrache, le pavot oeillette, la carthame, le chénevis, la courge, le ricin, le sésame, la sariette, le souci et le thym.

La « plante » selon l'invention peut être choisie parmi toutes les espèces de plantes cultivées listées ci-dessus, et particulièrement parmi les différentes variétés de ces espèces.

Préférablement, les « plantes » selon l'invention sont les plantes de grandes cultures, les cultures légumières (dont hors sol) et la viticulture, telles que définies ci-dessus.

L'application peut être effectuée à n'importe quel stade de développement de la plante. L'application peut par exemple être effectuée directement sur les semences (par exemple par enrobage juste avant le semis ou avec des semences pré-enrobées avec comme base d'enrobage des mélanges de Carboxyméthyl-cellulose et d'amidon), sur les sols à cultiver ou les supports de culture liquide ou solide (avant ou après le semis), par exemple par distribution de l'inoculum dans la ligne de semis (par exemple en mélangeant l'inoculum avec des micro-granulés d'argile distribués dans la ligne de semis ou par inoculation directe sur semences à l'aide de liquides adhésifs), directement sur la plante par pulvérisation foliaire ou directement sur un explant végétal ou sur une bouture.

Typiquement, lorsqu'elle est appliquée directement sur les semences, la composition est mélangée avec les semences à la main, dans un tambour rotatif ou dans un mélangeur. Ce mélange peut nécessiter l'ajout d'adhésifs, tels que la gomme arabique, le caboxyméthylcellulose, une solution de sucrose ou des huiles végétales, afin de s'assurer que les graines sont recouvertes d'un nombre nécessaire de bactéries (voir par exemple Senoo K, Narumi I, 2006. Carrier materials. p. 41-49; BiofertilizerManual. Japan Atomic Industrial Forum (JAIF)). Après ce mélange, les graines sont généralement séchées. Alternativement, la composition peut être pulvérisée sur les graines. Les graines ainsi pulvérisées sont ensuite semées après séchage.

Typiquement, lorsque la composition est appliquée directement sur le sol à cultiver, elle est sous forme de granulés et distribuée dans la ligne de semis. Alternativement, lorsqu'elle est sous forme liquide, la composition peut être pulvérisée sur le sol à cultiver.

Alternativement, lorsque le milieu de culture est un support pour la culture en hors-sol, comme par exemple l'aéroponie, l'hydroponie, ou l'aquaponie, la composition peut directement inoculée dans le substrat de culture, par exemple sous forme de tourbe.

Typiquement, l'application se fera au semis (par application sur le sol ou par enrobage des semences) ou par inoculation des substrats de culture pour les cultures en hors sol.

Selon un autre mode de réalisation, la présente invention porte également sur l'utilisation de la souche bactérienne déposée le 24 octobre 2018 à la Collection Nationale de Culture de Micro-organismes (CNCM), 28 rue du Dr. Roux, 75724 PARIS CEDEX 15, en vertu du traité de Budapest sous le numéro CNCM I-5372 en tant que biofertilisant.

L'invention porte également sur l'utilisation de la souche bactérienne déposée le 24 octobre 2018 à la Collection Nationale de Culture de Micro-organismes (CNCM), 28 rue du Dr. Roux, 75724 PARIS CEDEX 15, en vertu du traité de Budapest sous le numéro CNCM I-5372 en tant que phytostimulant.

Les exemples qui suivent illustrent plus en détail l'invention mais ne doivent pas être interprétés comme limitant sa portée.

### Brève description des dessins

**[****Fig. 1****]**

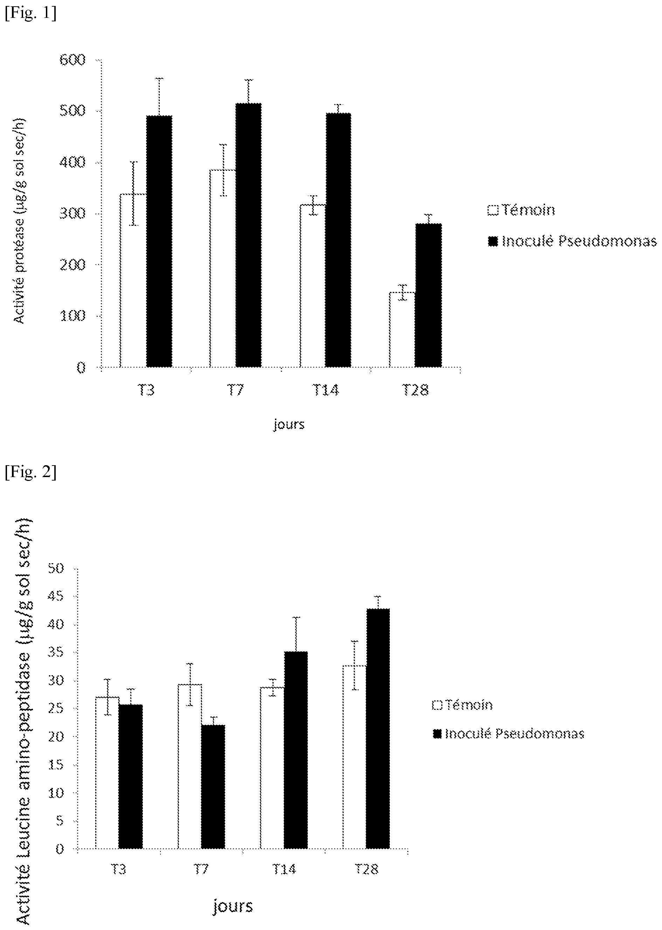

   [Fig. 1] représente l'activité protéase du sol après inoculation par la souche selon l'invention;
**[****Fig. 2****]**
   [Fig. 2] représente l'activité aminopeptidase du sol après inoculation par la souche selon l'invention;
**[****Fig. 3****]**

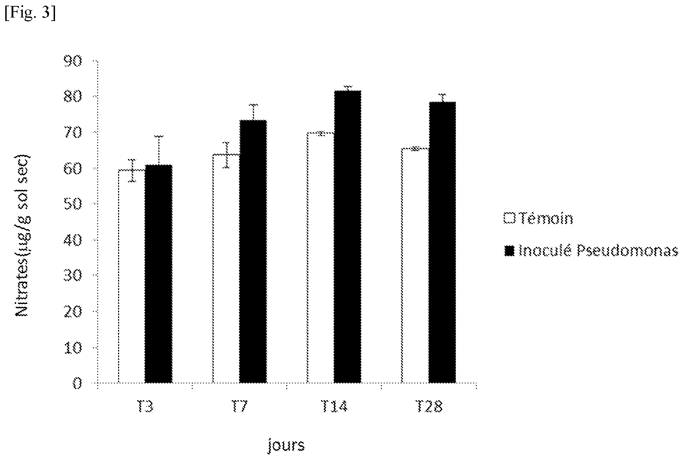

   [Fig. 3] représente la teneur en nitrates du sol après inoculation par la souche selon l'invention ;

### Exemples

### Exemple 1 : Analyse des propriétés biofertilisantes de la souche d'intérêt sur microcosme de sol (conditions contrôlées)

**Matériel et méthodes** : Un sol « modèle » a été prélevé sur une parcelle agricole de la ferme expérimentale de l'ENSAIA. Le sol (limon-argileux ; MO 4,5% ; N total 0,3 % ; C/N 13,8 ; pHₑₐᵤ 6,5) a été tamisé à 5 mm puis conservé à température ambiante jusqu'à utilisation. Des microcosmes de sol ont été réalisés en plaçant 70g de sol à 60% de sa capacité de rétention en eau dans des pots Le Parfait de 0.5L. Les microcosmes ont été pré-incubés pendant 2 semaines à 20°C puis ont été inoculés avec 5 ml de suspension bactérienne à 7^{∗}10⁹ UCF (soit 10⁸ UFC/g de sol frais) de la souche bactérienne sélectionnée. Les microcosmes témoins ont été traités avec 5 ml d'eau physiologique stérile. Les microcosmes de sol, à 80% de la capacité de rétention en eau, ont alors été incubés à 20°C à l'obscurité. Des prélèvements de sol ont été réalisés 0, 3, 7, 14 et 28 jours après inoculation. Quatre répétitions par traitement et temps de prélèvement ont été réalisées. A chaque temps de prélèvement, un ensemble de variables ont été mesurées dans les 48h suivant le prélèvement, à savoir les (i) activités enzymatiques microbiennes du sol en lien avec la dynamique de N, S et P, (ii) les mesures des teneurs en C et N solubles dans l'eau, (iii) les biomasses microbiennes C et N, (iv) les teneurs en N, S et P minéral et (v) les teneurs en acides aminés des sols. Des échantillons de sol ont été immédiatement congelés à -20°C pour les extractions d'ADN et la mesure de l'abondance des gènes ADNr 16S.

L'activité potentielle arylsulfatase dans le sol a été mesurée d'après le protocole de Tabatabai et Bremner (1970). L'activité potentielle protéase a été mesurée d'après le protocole de Ladd et Butler (1972). L'activité potentielle phosphatase a été mesurée d'après le protocole de Dick et al. (1996). Enfin l'activité potentielle leucine-aminopeptidase a été mesurée d'après le protocole de Spungin et Blumberg (1989).

Afin d'estimer les teneurs en N organique soluble et en N minéral des sols, 10 g de sol frais ont été extraits avec 50 ml de KCL 1M pendant 45 min à l'aide d'un agitateur révolutionnaire. Les extraits ont été filtrés sur filtre Whatman n°42 (Harper, 1924) puis conservés à -20°C jusqu'à analyse. Les teneurs en N minéral (NO₃⁻ et NH₄⁺) des extraits ont été déterminées en utilisant un spectrophotomètre d'absorption moléculaire SAN++ CFA (Skalar Analytical, Breda, Pays-Bas) par la plateforme d'analyses de l'INRA de Nancy. Les acides aminés ont été quantifiés d'après le protocole de Darrouzet-Nardi et al. (2013). Le C et le N soluble ont été extraits à l'eau chaude comme décrit par Vong et al. (2007). La teneur en C et N des extraits à l'eau chaude a été déterminée avec un TOC-V CHS (Shimadzu, Kyoto, Japon).

Les SO₄²⁻ ont été extraits du sol par agitation révolutionnaire de 10g de sol dans 50 ml de KH₂PO₄ 16mM pendant 45 min (Walker et Doornenbal, 1972) puis filtrés une première fois sur papier Whatman n°42 et une deuxième fois sur filtre de 0,45 µm de porosité. Les SO₄²⁻ des extraits KH₂PO₄ ont été par la suite dosés par chromatographie ionique (Dionex).

Les PO₄²⁻ ont été extraits par agitation révolutionnaire de 5 g de sol dans 50 ml de NaHCO₃ 0,5M pendant 45 min puis filtrés sur papier Whatman n°42 d'après le protocole de Hedley et al. (1982). Les PO₄²⁻des extraits NaHCO₃ ont été par la suite dosés d'après le protocole de Irving et McLaughlin (1990).

Les biomasses microbiennes C et N ont été mesurées par la méthode de fumigation-extraction (Vance et al., 1987). Les concentrations en C et N des extraits au K₂SO₄ 0.5M fumigés et non fumigés filtrés sur Whatman n°42 ont été déterminés avec un TOC-V CHS (Shimadzu, Kyoto, Japon). Les biomasses microbiennes ont été déterminées par différence entre les extraits fumigés et non fumigés et division par un coefficient d'extraction de 0,45 pour la biomasse microbienne C (Vance et al., 1987) et de 0,54 pour la biomasse microbienne N (Brookes et al., 1985).

Résultats : En conditions contrôlées, la souche affiliée à *Pseudomonas* (souche P) stimule certaines activités microbiennes du sol impliquées dans la minéralisation des matières organiques, sans modifier la taille de la biomasse microbienne du sol. Ainsi, les activités microbiennes en lien avec la décomposition de l'azote sont significativement plus élevées dans les sols inoculés comparativement aux sols non inoculés. Sur 28 jours d'incubation, l'activité protéase qui participe à la décomposition des protéines, principale forme de N organique dans les sols, est en moyenne 40% supérieure dans les sols inoculés avec la souche P comparativement aux sols non inoculés (Fig. 1). L'activité leucine aminopeptidase qui dégrade les peptides libérés par les protéases en acides aminés est quant à elle augmentée de 22 % à 32 % dans les sols inoculés avec la souche P à partir de 14 jours après inoculation (Fig. 2). Les activités arylsulfatases qui minéralisent les esters de sulfate, forme majeure et parmi la plus labile de S organique dans les sols sont également stimulées dans les sols inoculés, faiblement 3 jours après inoculation (+ 15% d'activité dans les sols inoculés avec la souche P) et fortement 14 jours après inoculation (+189% d'activité dans les sols inoculés avec la souche P). Enfin, les activités phosphatases ne sont que faiblement et transitoirement stimulées 28 jours après inoculation des sols avec la souche P (+8% comparativement au sol non inoculés).

La souche P améliore la disponibilité en N minéral sous forme nitrates du sol, les nitrates constituant la principale source de N pour la nutrition des plantes. Les teneurs en nitrates des sols inoculés avec la souche P sont augmentés de 15 à 20% de 7 à 28 jours après inoculation, soit une augmentation moyenne de + 11,5 mg de nitrates /kg de sol (Fig. 3). Ceci représente un potentiel d'environ 45 unités de N/ha.

### Exemple 2 : Analyse des propriétés phytostimulantes de la souche d'intérêt (conditions contrôlées)

**Matériel et méthodes** : Une première expérimentation en conditions gnotobiotiques a été mise en place afin d'étudier l'effet de la souche bactérienne P sur la croissance et l'architecture racinaire de plantules de maïs (Pioneer Hi Bred). Les semences utilisées ont été stérilisées en surface. Pour cela, les semences ont été placées successivement en présence d'éthanol à 70% pendant 5 min et d'hypochlorite de sodium à 5% pendant 10 min. Les semences ont alors été rincées 5 fois avec de l'eau distillée stérile. 100µl de la dernière eau de rinçage ont été étalés sur un milieu gélosé NB afin de contrôler l'efficacité de la stérilisation de surface.

Environ 25 semences stérilisées ont alors été mises à germer dans des boîtes de Pétri de 13,5 cm de diamètre, sur papier filtre Whatman n°3 stérile et humidifié avec 10 ml d'eau distillée stérile. Les boîtes de Pétri ont été placées à l'obscurité pendant 3 jours à 28°C. Quatre semences pré-germées ont alors été sélectionnées et disposées sur une ligne équatoriale dans une boîte de Pétri de 13,5 cm de diamètre. Chaque semence a été inoculée avec 100µl de suspension bactérienne (10⁹ bactéries/semence) de la souche sélectionnée. L'inoculum bactérien a été préparé à partir d'une culture à 10⁹ UFC/ml. Un ml de culture a été centrifugé à 10 000 rpm pendant 10 min puis le culot a été repris dans 100 µl d'eau physiologique stérile. Les semences pré-germées témoins ont reçu 100 µl d'eau physiologique stérile. Au total, 4 répétitions ont été réalisées par traitement. Les boîtes de Pétri ont été placées dans une enceinte climatique avec une photopériode de 16h, une température de 23°C jour et 18°C nuit pendant 6 jours. Après incubation, les parties racinaires ont été collectées. Un ensemble de traits racinaires ont été analysés. Pour cela, le système racinaire a été étalé dans une fine couche d'eau, dans une cuve de plexiglas (1,5* 20*30 cm), à l'aide de pinces fines. Le système racinaire a alors été scanné (scanner Expression 1640XL, Epson) et les images obtenues ont été analysées avec le logiciel WinRhizo^{©} (Régent Instruments Inc., Québec, Canada). La longueur totale (cm), la longueur des racines fines (diamètre < 2mm) et grossières (diamètre > 2mm), la surface totale (cm²), la surface des racines fines et grossières le diamètre moyen (mm) des racines ont été estimés. Après analyse, les racines ont été séchées délicatement sur papier filtre puis pesées pour estimer la masse fraîche. Elles ont ensuite été placées à l'étuve à 80°C pendant 48h afin de déterminer la masse sèche.

Une seconde expérimentation a visé à évaluer l'effet de la souche P sur la croissance, la physiologie et le statut nutritionnel du maïs lors des premiers stades de développement ainsi que sur le fonctionnement biologique et la teneur en éléments minéraux dans la rhizosphère du maïs. Le sol (limon-argileux ; MO 1,6 % ; N total 0,12 % ; C/N 7,8 ; pHₑₐᵤ 7,5) a été prélevé sur une parcelle agricole (Saint Martin des champs, 77), séché à l'air libre puis tamisé à 5 mm. Avant utilisation, le sol a été mélangé avec 10% (m/m) de sable d'aquarium afin de faciliter la collecte ultérieure des racines.

L'inoculation des maïs avec les souches bactériennes d'intérêt a été réalisée au moment du semis, sur semences préalablement stérilisées et germées avec 1 ml d'un inoculum à une concentration de 10⁸ UFC/ml. Des semences stérilisées, germées mais non inoculées ont constitué le témoin. Une semence a été placée à une profondeur d'environ 2 cm dans un tube en PVC (5*20 cm) contenant 330 g du mélange sol-sable. Ce substrat a été maintenu à 80% de sa capacité de rétention en eau, en pesant les tubes 5 fois par semaine et en arrosant, si nécessaire, avec de l'eau. Aux stades 1-2 feuilles, 3-4 feuilles, 5 feuilles et 5-6 feuilles, 4 tubes de chaque modalité de traitement ont été choisis aléatoirement et ouverts à la scie circulaire. Après ouverture des tubes, les parties aériennes ont été séparées du système sol-racines et collectées. Les racines ont été séparées du sol, collectées à l'aide d'une pince à épiler puis lavées à l'eau du robinet. Les parties aériennes et racinaires des plantes et le sol ont été conservés à 4°C jusqu'à analyse de l'ensemble des variables. Un aliquote de sol a été congelé à -20°C pour les analyses moléculaires ultérieures.

Au niveau des plantes, la masse fraîche et la masse sèche des parties aériennes et racinaires ont été mesurées. L'architecture racinaire a également été analysée comme décrit précédemment (expérimentation en conditions gnotobiotiques). Au niveau du sol rhizosphérique du maïs, les variables d'abondance microbienne, d'activités enzymatiques microbiennes et de teneur en éléments minéraux ont été mesurées comme décrit dans le cadre de l'expérimentation en microcosmes de sol.

Résultats : En conditions gnotobiotiques (première expérimentation), les résultats obtenus mettent en évidence un effet significatif de l'inoculation de la souche P affiliée à *Pseudomonas* sur différentes variables racinaires. Ainsi, 6 jours après inoculation, la biomasse racinaire fraîche des plantules inoculées tend à être 1,5 fois plus élevée que celle des plantes témoin Ces effets sur la biomasse racinaire résultent principalement d'une augmentation significative de la longueur et de la surface des racines fines (+64% et + 58% respectivement), qui sont les racines considérées comme principalement impliquées dans l'absorption des nutriments par la plante (Eissenstat, 1992).

Dans la seconde expérimentation, nous avons analysé l'effet de la souche P sur la croissance et le statut nutritionnel du maïs cultivé en conditions contrôlées jusqu'au stade 6 feuilles ainsi que le fonctionnement biologique et la disponibilité en éléments minéraux dans la rhizosphère de ce maïs.

Au niveau des plantes, si la souche n'influence pas la biomasse des parties aériennes du maïs ni leur hauteur, elle modifie la croissance racinaire. Ainsi, la biomasse fraîche des racines des plantes inoculées avec la souche P tend à être supérieure à celle des plantes non inoculées au stade 1-2 feuilles. Des modifications au niveau de l'architecture racinaire des plantes inoculées avec la souche affiliée à Pseudomonas (souche P) sont observées au stade 1-2 feuilles. Ainsi, la surface (+15%, p=0,03) des racines fines (diamètre < à 2mm) est plus importante pour les plantes inoculées en comparaison du témoin. Ces résultats tendent à confirmer les résultats obtenus en conditions gnotobiotiques.

Concernant les effets de l'inoculation de la souche P sur le fonctionnement du sol rhizosphérique, des mesures d'abondance microbienne, d'activités enzymatiques microbiennes impliquées dans la dynamique de N, S et P et de teneurs en éléments minéraux ont été réalisées. Les principaux effets de l'inoculation de la souche P sur les activités enzymatiques du sol en lien avec les cycles N, S et P sont observés au stade 3-4 feuilles du maïs. Ainsi, l'activité protéase dans la rhizosphère des plants de maïs inoculés avec la bactérie P affiliée à *Pseudomonas* est en moyenne 92% supérieure (p=0,02) à celle dans le sol témoin. Dans ces mêmes sols, les activités leucine-aminopeptidases sont également significativement supérieures à celles des sols témoins (+57%). L'activité arylsulfatase impliquée dans la minéralisation de S est significativement augmentée dans la rhizosphère des plantes inoculées avec la souche P (+101%) comparativement au témoin au stade 3-4 feuilles. Au stade 5-6 feuilles, cette activité reste plus élevée dans les sols inoculés (+21%, pour la souche P).

### Exemple 3 : Utilisation de marqueurs moléculaires pour discriminer la souche P

La possibilité de discriminer des souches bactériennes avec des marqueurs de type RAPD a été validée en comparant le profil de la souche P à d'autres souches notamment du genre Pseudomonas (très représenté dans les environnements naturels).

La liste des souches testées est listée dans le tableau ci-après :

**Tableau 1 : Liste des souches testées**

| **Code souche** | **Nom souche** |
|---|---|
| Achromo | *Achromobacter sp.* |
| Ps.chloro | *Pseudomonas chlororaphis* |
| Ps.sp | *Pseudomonas sp.* |
| Ps.putida | *Pseudomonas putida* |
| Enterob | *Enterobacterludwigii* |
| Ps-1 | *Pseudomonas moraviensis* (souche P) |
| Ps-2 | *Pseudomonas moraviensis* (souche P) |
| Micro-1 | *Microbacterium resistens* |
| Micro-2 | *Microbacterium resistens* |

Deux marqueurs de type RAPD (Random Amplified Polymorphic DNA) ont été testés: M13 5'-GAGGGTGGCGGTTCT-3' (SEQ ID NO :1) et RAPD2 5'-AGCAGCGTGG 3' (SEQ ID NO :2). Les réactions d'amplification ont été réalisées dans un volume final de 12.5µL en présence de 15 ng d'ADN et de 25µM d'amorces. Les amplicons sont séparés sur gel d'agarose à 2 % afin de révéler les profils.

Les deux marqueurs sont discriminants même vis-à-vis de bactéries du même genre (i.e. Pseudomonas). On observe une meilleure répétabilité pour le marqueur M13.

### Exemple 4 : Production de la souche P

Le scale-up a été réalisé dans un bioréacteur de type Applikon^{®} dans un volume final de 4L. Le milieu utilisé est composé de 10g/L de lait entier en poudre et de 5g/L d'extrait de levure. Les essais permettront de déterminer le kLa (coefficient de transfert d'oxygène), paramètre de la montée en échelle (bioréacteurs 50 puis 500 L). La température optimale est de 30 °C (voir tableau ci-dessous).

**Tableau 2 : Conditions de production de la souche P**

| Paramètre | Aération | Agitation | pH | Température |
|---|---|---|---|---|
| *P. moraviensis* souche P | 2 vvm | 150 rpm | 7.3 - 7.5 | 30°C |

Les essais conduits dans les fermenteurs 5L ont permis de produire de la biomasse microbienne de la souche P à une moyenne de 3.93^{E+09} UFC/ml.

### Exemple 5 : Séchage de la souche P

La souche P présente une très bonne survie au processus de lyophilisation pour les conditions avec cryoprotectant, encore plus marqué pour la condition avec saccharose. Les barèmes de lyophilisation sont présentés dans le tableau 3. De plus, la congélation des échantillons en vue de la lyophilisation n'a pas eu d'impact sur la viabilité des bactéries. L'aw des échantillons est au maximum d'environ 0,150 et permet de garantir une bonne stabilité microbiologique (viabilité et conservation). La durée totale de la lyophilisation est de 47H.

**Tableau 3 : Barèmes de lyophilisation**

| **Etape** | **Température finale des étagères (°C)** | **Rampe (min)** | **Pression (µbar)** | **Durée du palier (min)** |
|---|---|---|---|---|
| **Congélation** | -45 | Avant chargement | Atmo | 120 |
| **Dessiccation Primaire** | -30 | 60 | 50 | 990 |
| | -10 | 150 | 50 | 1350 |
| | 10 | 120 | 50 | 300 |
| **Dessiccation Secondaire** | 20 | 90 | 50 | 60 |

Avec cryoprotectant, la perte de viabilité est négligeable. On observe donc un effet positif du cryoptotectant (saccharose à 5 %, tableau 4).

**Tableau 4 : Viabilité des souches**

| **Condition** | **Viabilité (CFU/mL) avant lyophilisation** | **Viabilité avant lyophilisation (logCFU/mL)** | **Viabilité avant lyophilisation (CFU/g MS)** | **Viabilité après lyophilisation (CFU/mL)** | **Viabilité après lyophilisation (logCFU/mL)** | **Perte Viabilité (réduction log)** |
|---|---|---|---|---|---|---|
| **Souche P sans Cryoprotectant** | 3.57E+10 | 10.55 | 6.93E+10 | 1.24E+09 | 9.09 | 1.46 |
| **Souche P avec Saccharose 5%** | 4.47E+10 | 10.65 | 8.04E+11 | 2.27E+10 | 10.36 | 0.29 |

## Revendications

1. Souche bactérienne déposée le 24 octobre 2018 à la Collection Nationale de Culture de Micro-organismes (CNCM), 28 rue du Dr. Roux, 75724 PARIS CEDEX 15, en vertu du traité de Budapest sous le numéro CNCM I-5372.

2. Composition comprenant la souche bactérienne selon la revendication 1, dans laquelle ladite composition est sous forme de poudre, de granulés, de crème ou sous forme liquide.

3. Composition selon la revendication 2, dans laquelle ladite composition est sous forme liquide.

4. Composition selon la revendication 3, dans laquelle ladite souche bactérienne est sous forme d'une suspension bactérienne dans une solution contenant de l'eau et/ou des huiles minérales et/ou des huiles organiques.

5. Procédé de fertilisation comprenant une étape dans laquelle la souche bactérienne selon la revendication 1, ou la composition selon l'une quelconque des revendications 2 à 4, est appliquée à une plante ou à un sol.

6. Procédé de fertilisation selon la revendication 5, dans lequel ladite plante est choisie parmi les plantes de grandes cultures, les plantes de cultures légumières et les plantes cultivées en viticulture.

7. Procédé de fertilisation selon la revendication 5 ou 6, dans lequel l'application est faite au semis, par application sur le sol ou par enrobage des semences.

8. Procédé de fertilisation selon la revendication 5 ou 6, dans lequel l'application est faite par inoculation des substrats de culture destinés à la culture en hors sol.

9. Utilisation de la souche bactérienne selon la revendication 1 en tant que biofertilisant.

10. Utilisation de la souche bactérienne selon la revendication 1 en tant que phytostimulant.

## Patentansprüche

1. Bakterienstamm, der am 24. Oktober 2018 bei der Collection Nationale de Culture de Micro-organismes (CNCM), 28 rue du Dr. Roux, 75724 PARIS CEDEX 15, gemäß dem Vertrag von Budapest unter der Nummer CNCM I-5372 hinterlegt wurde.

2. Zusammensetzung, umfassend den Bakterienstamm nach Anspruch 1, wobei die Zusammensetzung in Form eines Pulvers, Granulats, einer Creme oder in flüssiger Form vorliegt.

3. Zusammensetzung nach Anspruch 2, wobei die Zusammensetzung in flüssiger Form vorliegt.

4. Zusammensetzung nach Anspruch 3, wobei der Bakterienstamm in Form einer Bakteriensuspension in einer Lösung vorliegt, die Wasser und/oder Mineralöle und/oder organische Öle enthält.

5. Düngeverfahren, umfassend einen Schritt, wobei der Bakterienstamm nach Anspruch 1 oder die Zusammensetzung nach einem der Ansprüche 2 bis 4 auf eine Pflanze oder einen Boden aufgebracht wird.

6. Düngeverfahren nach Anspruch 5, wobei die Pflanze aus Ackerbaupflanzen, Gemüseanbaupflanzen und Weinbaupflanzen gewählt wird.

7. Düngeverfahren nach Anspruch 5 oder 6, wobei die Anwendung bei der Aussaat, durch Aufbringen auf den Boden oder durch Umhüllen des Saatguts erfolgt.

8. Düngeverfahren nach Anspruch 5 oder 6, wobei die Anwendung durch Impfung von Kultursubstraten für den bodenlosen Anbau erfolgt.

9. Verwendung des Bakterienstamms nach Anspruch 1 als Bio-Düngemittel.

10. Verwendung des Bakterienstamms nach Anspruch 1 als Phytostimulans.

## Claims

1. A bacterial strain deposited on October 24, 2018, at the *Collection Nationale de Culture de Microorganismes* (CNCM), 28 rue du Dr. Roux, 75724 PARIS CEDEX 15, under the Budapest Treaty under number CNCM I-5372.

2. A composition comprising the bacterial strain according to claim 1, wherein said composition is in powder, granule, cream or liquid form.

3. The composition according to claim 2, wherein said composition is in liquid form.

4. The composition according to claim 3, wherein said bacterial strain is in the form of a bacterial suspension in a solution containing water and/or mineral oils and/or organic oils.

5. A fertilization process comprising a step in which the bacterial strain according to claim 1, or the composition according to any one of claims 2 to 4, is applied to a plant or to a soil.

6. The fertilization process according to claim 5, wherein said plant is selected from field crop plants, vegetable crop plants and plants grown in viticulture.

7. The fertilization process according to claim 5 or 6, wherein the application is made at sowing time, by application to the soil or by coating the seeds.

8. The fertilization process according to claim 5 or 6, wherein the application is made by inoculation of growing substrates intended for soilless cultivation.

9. Use of the bacterial strain according to claim 1 as biofertilizer.

10. Use of the bacterial strain according to claim 1 as phytostimulant.
